# FASCICULE DE BREVET EUROPEEN

(11) **EP 2 152 157 B1**
(45) Date de publication et mention de la délivrance du brevet: **13.04.2016**
(21) Numéro de dépôt: 08805705.4
(22) Date de dépôt: 25.04.2008
(51) Int. Cl.: A61B 5/04, A61B 5/055

(54) **PROCÉDÉ, DISPOSITIF ET SYSTÈME POUR RÉDUIRE LES ARTEFACTS AFFECTANT LES SIGNAUX ÉLECTROPHYSIOLOGIQUES ET DUS AUX CHAMPS ÉLECTROMAGNÉTIQUES**
VERFAHREN, VORRICHTUNG UND SYSTEM ZUR REDUZIERUNG VON ARTEFAKTEN, DIE ELEKTROPHYSIOLOGISCHE SIGNALE BEEINTRÄCHTIGEN UND VON ELEKTROMAGNETISCHEN FELDERN VERURSACHT WERDEN
METHOD, DEVICE AND SYSTEM FOR REDUCING ARTIFACTS AFFECTING ELECTROPHYSIOLOGICAL SIGNALS AND CAUSED BY ELECTROMAGNETIC FIELDS

(30) Priorité: 27.04.2007 FR 0754747
(43) Date de publication de la demande: 17.02.2010
(73) Titulaire: Schiller Medical (société Par Actions Simplifiée), 67160 Wissembourg (FR); Universite Henri Poincare-Nancy I (Etablissement Public à Caractère Scientifique, Culturel et Professionnel), 54000 Nancy (FR); Université de Strasbourg (Etablissement Public National à Caractère Scientifique, Culturel et Professionnel), 67000 Strasbourg (FR); Centre Hospitalier Et Universitaire De Nancy (Etablissement Public de Santé), 54000 Nancy (FR)
(72) Inventeur: FELBLINGER, Jacques, F-54850 Mereville (FR); KRAEMER, Michel, F-67360 Durrenbach (FR); BLONDE, Jean-Philippe, F-67460 Souffelweyersheim (FR); ABÄCHERLI, Roger, CH-6340 Baar (CH); FRICK, Vincent, F-67000 Strasbourg (FR); SCHMID, Johann-Jakob, CH-8911 Rifferswil (CH)
(74) Mandataire: Nuss, Laurent
(86) Numéro de dépôt international: PCT/FR2008/050751
(87) Numéro de publication internationale: WO 2008/155488

(56) Documents cités:
- EP-A- 1 050 270
- EP-A1- 0 721 110
- WO-A-2006/015938
- WO-A-2007/073576
- US-A- 5 445 162
- US-A1- 2005 277 826

## Description

La présente invention concerne un procédé, un dispositif capteur et un système ayant pour objectif le recueil d'au moins un signal électrophysiologique pour sa visualisation, son impression, son analyse et son interprétation dans le cadre de la surveillance du patient sur lequel il est recueilli et pour l'activation de fonctions secondaires.

Ces fonctions secondaires peuvent être la synchronisation prospective ou rétrospective d'un imageur (par exemple Imagerie par Résonance Magnétique, Tomographie par Emission de Positron, Tomodensitométrie par rayons X, Scintigraphie, Echographie).

Un exemple de dispositif et de système remplissant cet objectif est le moniteur de la demanderesse connu sous la désignation Maglife C, qui recueille l'électrocardiogramme dans un environnement IRM.

L'imagerie par résonance magnétique permet des examens non invasifs en routine clinique. Cette technique utilise un aimant de forte valeur, principalement sous forme d'un tunnel, dans lequel le patient est allongé. Pour produire les images, des gradients de champs magnétiques et des impulsions radiofréquences sont utilisés au cours de séquences choisies en fonction des images recherchées.

Les signaux électrophysiologiques généralement concernés dans le cadre de la présente invention sont l'ElectroCardioGramme (ECG), l'ElectroEncéphaloGramme (EEG), l'ElectroMyogramme (EMG) et l'ElectroOcculogramme (EOG). Ces signaux peuvent être utilisés pendant des examens IRM soit pour une surveillance du patient ("Monitoring"), soit pour la synchronisation des séquences IRM ou encore pour des buts de recherche.

Ces signaux électriques sont malheureusement perturbés par l'environnement IRM pendant la génération des images. A ce sujet, on peut par exemple citer les publications suivantes :
J. Felblinger, C. Lehmann, C. Boesch, "Electrocardiogram acquisition during MR examinations for patient monitoring and sequence triggering", Magnetic Resonance in Medicine, 32, 523-529 (1994) ;
J. Felblinger, J. Debatin, C. Boesch, R. Gruetter, G. McKinnon, "Synchronisation device for ECG-gated echo planar imaging of the human heart", Radiology, 197, 311-313 (1995) ;
J. Felblinger, R.M. Mueri, C. Ozdoba, G. Schroth, C.W. Hess, C. Boesch, "Recording of eye movements for stimulus control during fMRI by means of electro-oculographic methods", Magnetic Resonance in Medicine, 36, 410-414 (1996) ;
R.M. Mueri, J. Felblinger, K.M. Roesler, B. Jung, C.W. Hess, C. Boesch, "Recording of electrical brain activity in a magnetic resonance environment: distorting effects of static magnetic field", Magnetic Resonance in Medicine, 39, 18-22 (1998).

L'utilisation de l'électrocardiogramme pour la synchronisation prospective ou rétrospective de l'imageur ne nécessite pas nécessairement une suppression parfaite des interférences sur le signal. Le signal de synchronisation peut être obtenu à partir de la combinaison et du traitement de plusieurs signaux électrocardiologiques recueillis simultanément (US-2006/173249).

Le document US-4 991 580 propose une méthode de détection de l'onde QRS (à partir de laquelle s'effectue le processus de synchronisation) basée sur la recherche et l'enregistrement d'une forme de cette onde avant l'examen IRM qui provoque ces interférences.

Il existe aussi des méthodes de synchronisation de la prise des images avec l'activité cardiaque qui ne sont pas basées sur le recueil de l'électrocardiogramme. Elles sont basées sur l'utilisation d'images réalisées par l'imageur lui-même pour obtenir des informations sur l'activité cardiaque avec laquelle la prise d'image doit être synchronisée. Ces techniques ne sont accessibles que sur des imageurs IRM récents et dédiés.

En ce qui concerne le recueil de l'électrocardiogramme dans un but de surveillance des patients, et donc débarrassé au maximum des interférences indésirables, différentes solutions ont été proposées au cours des dernières années.

Des méthodes très simples consistent à torsader les fils d'acquisition (Wendt, 1988), à appliquer des filtres variables en fonction de la présence ou l'absence d'émissions de gradients de champs magnétiques (Rokey, 1988).

Ces méthodes sont devenues insuffisantes du fait de l'évolution de la technologie de l'IRM, en particulier avec l'augmentation de l'amplitude, la vitesse de commutation et la fréquence de répétition des gradients de champs.

Un premier saut technologique (US-5 782 241) a consisté à placer toute l'électronique d'amplification à proximité du point de mesure et à utiliser une transmission optique ou RF pour transmettre le signal en dehors de l'aimant de l'imageur. Cette solution a permis une avancée importante pour la réduction des interférences sur les signaux électrophysiologiques et présente une méthode particulièrement adaptée de transmission, puisqu'une liaison optique est insensible aux champs électromagnétiques et ne peut entraîner des risques d'échauffement et de brûlures.

Une gamme de solutions basées sur l'apprentissage de l'effet des gradients de champs magnétiques sur les signaux électrophysiologiques est apparue.

Laudon (1998) utilise une boucle conductrice proche de l'endroit de mesure de l'ECG pour recueillir des signaux identiques aux interférences qui se superposent au signal électrocardiologique pour pouvoir l'en débarrasser (soustraction). Cette approche ne donne pas entière satisfaction car il a été montré que la plus grande partie des artéfacts est générée dans les tissus, ce que la boucle conductrice ne peut pas mesurer (Felblinger, 1994).

Par le brevet US-6 873 869 (ou EP 1 050 270), il a été proposé un procédé qui consiste à mesurer simultanément deux signaux électrophysiologiques, l'un à l'aide de deux électrodes placées sur le thorax, approximativement de part et d'autre du coeur, et l'autre à l'aide de deux électrodes placées symétriquement aux précédentes par rapport à la ligne médiane du thorax, sur la même ligne horizontale. Le premier ensemble d'électrodes mesure principalement le signal électrophysiologique ECG avec les interférences et le deuxième mesure principalement les interférences. En effectuant une soustraction de ces deux signaux, il a été possible de réduire les interférences dans de nombreux cas.

Toutefois, les deux mesures ne sont pas réalisées au même endroit, d'où il résulte une prise en compte des interférences au niveau d'une zone distincte de la zone de mesure. En outre, le second ensemble d'électrodes ne mesure pas uniquement les interférences, mais également une partie du signal utile, et pas conséquent sa soustraction du signal fourni par le premier ensemble d'électrodes ne permet pas d'aboutir de manière sûre à un signal électrophysiologique non diminué et non perturbé.

Une amélioration de ce procédé, qui est basé sur l'estimation des interférences uniquement à partir des signaux électrophysiologiques, peut être obtenue par l'utilisation de méthodes de traitement de signal plus sophistiquées (par exemple par filtrage adaptatif, analyse en composantes principales ou indépendantes ...).

Allen (2000) propose une méthode basée sur la suppression de l'artefact moyen calculé pendant une séquence d'apprentissage. Cette méthode se base sur le fait que l'artéfact est identique durant toute la séquence, ce qui est généralement le cas pour l'IRM fonctionnelle mais ne peut pas être généralisé en IRM.

Sijbers (1999) utilise les données de la séquence IRM appliquée. Cette solution n'est possible que sur des systèmes de recherche pour lesquels les séquences IRM mises en oeuvre sont totalement connues.

Différentes autres techniques de traitement de signal ont été développées. Elles sont toutes basées sur la connaissance des courants de génération des gradients de champs magnétiques. Felblinger (1999) propose de calculer la réponse impulsionnelle du système à des gradients de champs magnétiques selon les trois directions, en utilisant une séquence spécifique. Avec ces réponses impulsionnelles, il est alors possible de corriger en temps réel les artéfacts produits pendant une séquence quelconque.

Une approche généralisée a été proposée par Odille (2006) en utilisant une méthode de déconvolution plus générale pour trouver les réponses impulsionnelles.

Toujours en utilisant les courants de génération des gradients de champs, Abächerli (2005) propose une approche adaptative pour trouver la réponse impulsionnelle pendant une séquence quelconque. Une méthode adaptative est également proposée par Kreger (US-6 675 036).

Ces dernières méthodes sont basées sur la connexion du système de traitement de signal à l'électronique de commande des gradients de l'IRM. En connaissant la source des interférences par la mesure des courants injectés dans les bobines de gradient, il est possible de déterminer à chaque instant les perturbations générées.

Cependant, ces méthodes présentent certaines difficultés et limitations de mise en oeuvre pratique. Ainsi, la connexion à l'électronique de commande des gradients pose différents problèmes, car il est nécessaire de faire une liaison directe entre le module de traitement de signal et l'annoire de l'IRM, ce qui n'est pas toujours aisément réalisable.

Par ailleurs, du fait des courants de Foucault indésirables induits, les courants des bobines de gradient ne correspondent pas exactement aux champs magnétiques locaux produits et mesurables, et donc pas non plus aux perturbations induites par ces champs. En effet, les courants dans les bobines tiennent compte de la compensation de ces courants de Foucault de manière à obtenir le champ désiré en tout point du volume appelé "Field of View" (champ de vue) qui est la zone possible pour l'imagerie.

De plus, si le capteur se trouve en dehors de cet espace dans lequel le champ varie linéairement (FOV ou Field of View - champ de vue), la valeur du courant des gradients ne correspond pas au champ réel proche du capteur.

La présente invention a pour but principal de proposer une solution permettant notamment de surmonter les difficultés et limitations précitées, en prenant en compte le facteur perturbateur réel local, et non sa génération, ni ses effets.

A cet effet, l'invention a pour objet un procédé de recueil avec un dispositif capteur d'au moins un signal physiologique d'un sujet vivant soumis à au moins un champ électromagnétique, en particulier soumis à un environnement électromagnétique tel qu'existant dans un appareil d'investigation exploitant la résonance magnétique, consistant à réaliser une acquisition physique d'au moins un signal électrophysiologique au niveau du ou sur le sujet, et au moins une opération de prétraitement dudit au moins un signal électrophysiologique prélevé, ce par l'intermédiaire de moyens correspondants, procédé caractérisé en ce qu'il consiste également à réaliser, par l'intermédiaire de moyens additionnels, une mesure d'au moins une caractéristique de l'environnement électromagnétique au niveau ou à proximité immédiate du ou des points ou zones d'acquisition du ou des signaux électrophysiologiques sur le sujet, et à corriger le ou chaque signal électrophysiologique SP relevé et prétraité, en fonction dudit au moins un signal de mesure SM, éventuellement prétraité, d'au moins une caractéristique de l'environnement électromagnétique du ou des points ou zones d'acquisition du signal SP précité.

Le principe à la base de la présente invention consiste, par conséquent, à la différence des procédés connus présentés ci-dessus, à mesurer, préférentiellement de manière continue, au moins une caractéristique du champ magnétique et les variations de celle-ci à l'endroit même du recueil du signal électrophyisiologique, perturbé par les variations dudit champ magnétique.

Le prétraitement dudit au moins un signal relevé peut notamment comprendre une ou plusieurs des opérations suivantes : amplification, filtrage, sélection de la ou des voies de traitement.

La caractéristique de l'environnement électromagnétique mesurée directement, au niveau ou à proximité immédiate du ou de chaque point d'acquisition du ou des signaux électrophysiologiques, peut être l'amplitude du vecteur champ magnétique local dans une ou plusieurs directions.

Une variante consiste à mesurer directement, au niveau ou à proximité immédiate du ou de chaque point d'acquisition du ou des signaux électrophysiologiques, la valeur du ou des gradients de champ magnétique présents, dans une ou plusieurs directions, ces gradients de champ étant les principaux perturbateurs et générateurs d'artéfacts pour les mesures de signaux électrophysiologiques en milieu IRM.

Selon une première variante de réalisation de l'invention, le procédé peut consister, en outre, à réaliser, avec des moyens adaptés, une modulation, une adaptation au mode de transmission et une émission vers une unité de gestion et de traitement distante, éventuellement associée à des moyens d'impression et/ou d'affichage, dudit au moins un signal électrophysiologique prétraité et dudit au moins un signal de mesure d'au moins une caractéristique de l'environnement électromagnétique du ou des points ou zones d'acquisition du signal précité, éventuellement également prétraité.

Selon une seconde variante de réalisation de l'invention, le procédé peut consister à réaliser le traitement correctif du ou de chaque signal électrophysiologique SP en fonction dudit au moins un signal de mesure SM à l'aide d'un moyen adapté intégré au dispositif capteur et ensuite à réaliser, avec des moyens adaptés, une modulation, une adaptation au mode de transmission et une émission vers une unité de gestion et de traitement distante, éventuellement associée à des moyens d'impression et/ou d'affichage dudit au moins un signal électrophysiologique corrigé.

L'invention concerne également un dispositif capteur d'au moins un signal physiologique d'un sujet vivant soumis à au moins un champ électromagnétique, en particulier soumis à un environnement électromagnétique tel qu'existant dans un appareil d'investigation exploitant la résonance magnétique, comprenant des moyens d'acquisition physique d'au moins un signal électrophysiologique sur le sujet, tels que des électrodes, et au moins des moyens de prétraitement dudit au moins un signal électrophysiologique prélevé, dispositif capteur caractérisé en ce qu'il comprend également des moyens additionnels pour la mesure d'au moins une caractéristique de l'environnement électromagnétique au niveau ou à proximité du ou des points ou zones d'acquisition du ou des signaux électrophysiologiques sur le sujet.

Les moyens additionnels précités comprennent avantageusement au moins un capteur de champ magnétique fournissant un signal de mesure fonction de l'amplitude du vecteur champ magnétique au niveau dudit capteur et au moins un moyen de prétraitement du signal délivré par le ou chaque capteur.

Ces moyens additionnels sont avantageusement logés dans le boîtier contenant les composantes du dispositif capteur, mais peuvent également être directement associés aux électrodes ou être logés dans un boîtier séparé.

Selon une variante constructive, le dispositif capteur comprend au moins une paire de capteurs de champ magnétique disposés de manière espacée suivant une direction déterminée, de manière à fournir la valeur d'au moins un gradient de champ selon une direction.

Selon un mode de réalisation pratique de l'invention, les moyens additionnels comprennent au moins un capteur de champ magnétique sous la forme d'un capteur à effet Hall pour mesurer l'amplitude du vecteur champ magnétique local ou, selon une variante, au moins une paire de capteurs à effet Hall disposés de manière espacée suivant une direction déterminée pour mesurer la valeur d'au moins un gradient de champ magnétique selon une direction.

En accord avec une première variante de réalisation, les signaux de mesure électrophysiologique(s) et magnétique(s), sont transmis séparément à partir du dispositif capteur et le traitement correctif des premiers signaux en fonction des seconds est réalisé à l'extérieur du dispositif capteur, par exemple par une unité de traitement située à distance dudit dispositif capteur, dans ou en dehors de l'enceinte renfermant l'appareil à résonance magnétique. Dans ce cas, le dispositif capteur comprend en outre des moyens de modulation, d'adaptation au mode de transmission et d'émission pour ledit au moins un signal électrophysiologique prétraité et pour ledit au moins un signal de mesure d'au moins une caractéristique de l'environnement électromagnétique du ou des points ou zones d'acquisition du signal précité, éventuellement également prétraité.

En accord avec une seconde variante de réalisation, la correction du ou des signaux électrophysiologiques est réalisée au niveau du dispositif capteur même. Dans ce cas, ce dernier comprend également, d'une part, un moyen de correction du ou de chaque signal électrophysiologique relevé et prétraité en fonction dudit au moins un signal de mesure, éventuellement prétraité, d'au moins une caractéristique de l'environnement électromagnétique du ou des points ou zones d'acquisition du signal précité, et, d'autre part, un moyen de modulation, d'adaptation au mode de transmission et d'émission pour ledit au moins un signal électrophysiologique corrigé.

Le dispositif capteur est adapté pour permettre de manière avantageuse la mise en oeuvre des variantes du procédé décrit précédemment.

Afin de réduire notamment au maximum la surface apparente du dispositif capteur soumise à l'influence de l'environnement électromagnétique et à ses variations, il peut être prévu que les moyens de prétraitement dudit au moins un signal électrophysiologique relevé et les moyens additionnels, ainsi qu'éventuellement les moyens de modulation et d'adaptation au mode d'émission et le moyen de correction, sont réalisés sous la forme de composants microélectroniques intégrés (de manière à éviter autant que possible toute perturbation du champ électromagnétique environnant), préférentiellement montés ou formés sur un circuit imprimé ou un substrat similaire, préférentiellement surmoulé ou monté dans un boîtier de protection adapté.

L'invention a également pour objet un système d'acquisition d'au moins un signal électrophysiologique d'un sujet vivant soumis à un examen par un appareil à RMN, en particulier à un examen d'IRM (imagerie par résonance magnétique), ce système comprenant notamment une unité de gestion et de traitement et au moins un dispositif capteur relié à ladite unité, ledit au moins un dispositif capteur étant positionné sur le sujet et l'unité de traitement étant située à distance, système caractérisé en ce que le ou chaque dispositif capteur est un dispositif capteur tel qu'évoqué ci-dessus et décrit plus en détail ci-après.

Ce système permettra notamment la mise en oeuvre du procédé décrit ci-dessus.

L'invention sera mieux comprise grâce à la description ci-après, qui se rapporte à des modes de réalisation préférés, donnés à titre d'exemples non limitatifs, et expliqués avec référence aux dessins schématiques annexés, dans lesquels :
la figure 1 est un schéma de principe simplifié d'un dispositif capteur selon l'invention ;
la figure 2A est un schéma de principe d'un système d'acquisition selon l'invention, comprenant un dispositif capteur ;
la figure 2B est un schéma de principe d'un système d'acquisition selon l'invention constituant une variante de réalisation équivalente, mais plus intégrée, de celui représenté sur la figure 2A, et

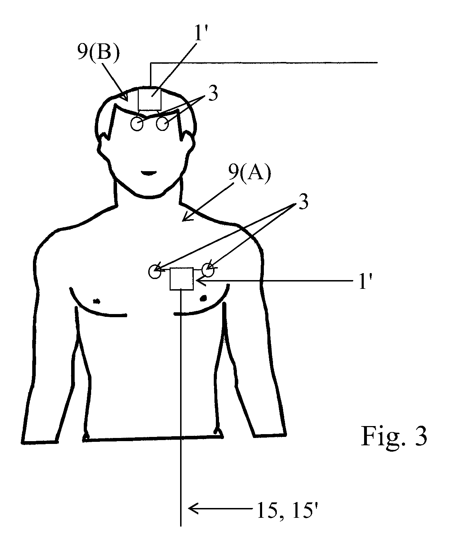

la figure 3 est une vue schématique partielle de dessus d'un sujet humain allongé illustrant des positionnements possibles pour les électrodes du dispositif capteur selon l'invention, pour relever des signaux d'électrocardiogramme 9(A) et pour relever un signal électroencéphalo-graphique 9(B).

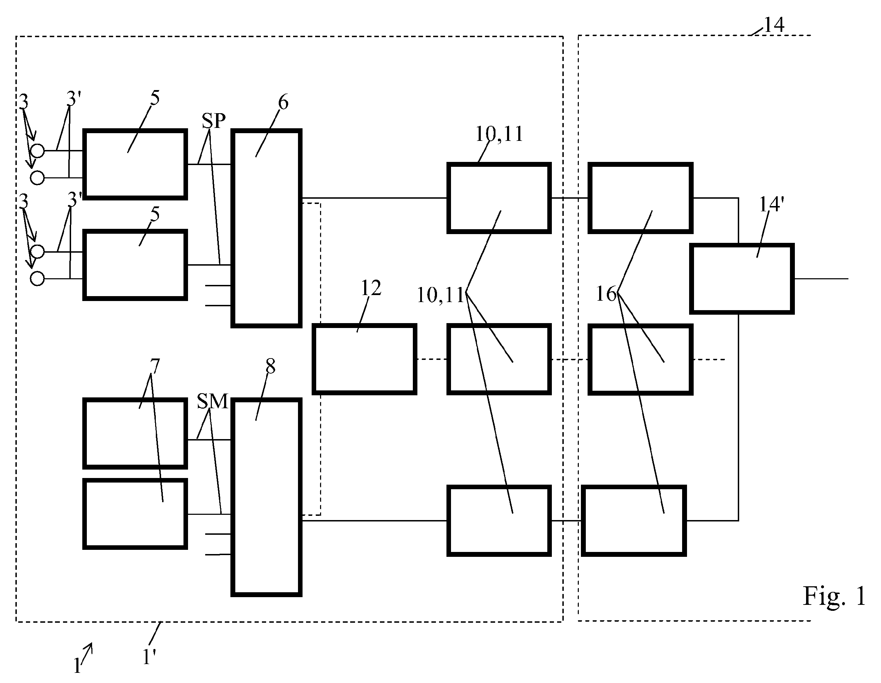

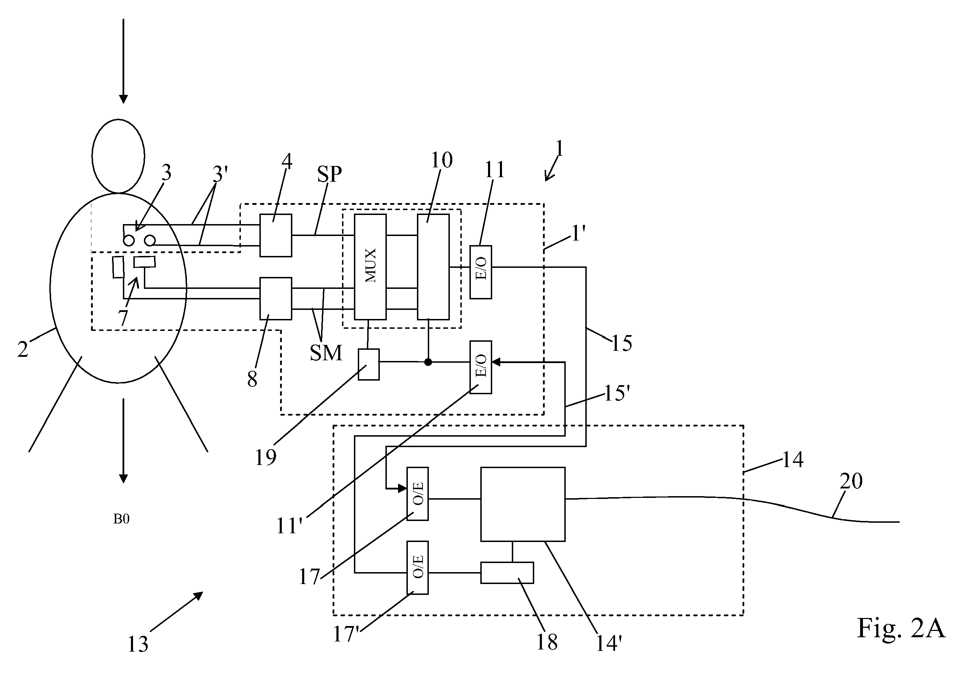

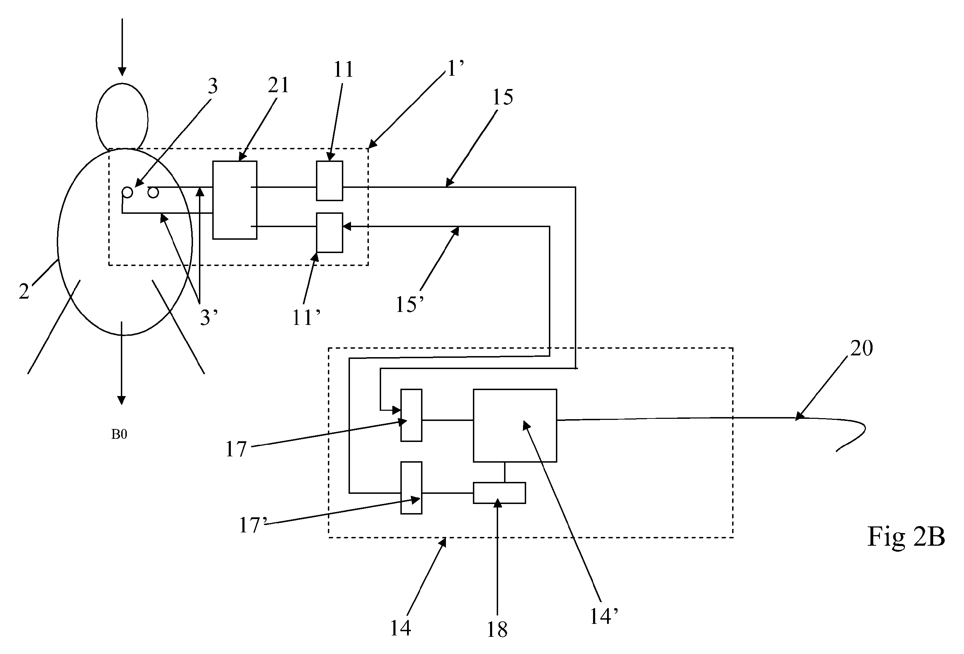

Comme le montrent les figures 1 et 2 des dessins annexés, le système d'acquisition 13 est principalement constitué de deux sous-ensembles, à savoir :
- un ou plusieurs dispositifs capteurs 1 (un seul est représenté) qui permet(tent) le recueil d'au moins un signal électrophysiologique SP (par exemple : EEG, ECG, ...) d'au moins un signal de mesure SM du champ magnétique local. Dans l'exemple de la figure 2, le patient est placé dans le tunnel de l'imageur et le champ B0 mesuré est parallèle au grand axe de ce tunnel.
- une unité 14 de traitement et de gestion placée à distance du patient, éventuellement en dehors de l'enceinte isolée renfermant l'appareil à résonance magnétique.

Les différents éléments constitutifs et les possibles structures et modes de fonctionnement peuvent être décrits de manière générale en relation avec la figure 1.

Comme le montre cette dernière, le dispositif capteur 1 comprend, du point de vue fonctionnel, essentiellement deux chaînes d'acquisition et au moins de prétraitement de signaux, à savoir :
- une chaîne dédiée aux signaux électrophysiologiques SP relevés sur le sujet 2, et
- une chaîne dédiée aux signaux SM représentatifs de l'environnement magnétique local, préférentiellement directement au niveau des points de mesure 9 des signaux électrophysiologiques SP.

Les capteurs 7 mesurant les caractéristiques magnétiques locales sont préférentiellement intégrés dans le boîtier de protection 1' du dispositif capteur 1, alors que les électrodes 3 relevant les signaux SP sont généralement reliées au dispositif capteur 1 par des conducteurs 3' de faible longueur, voire montées en sous-face dudit boîtier 1'.

La chaîne dédiée aux signaux électrophysiologiques SP peut comprendre plusieurs voies (deux voies sont représentées sur la figure 1), et chaque voie de recueil d'un signal électrophysiologique se compose d'au moins deux électrodes 3 connectées à un amplificateur de signal 5 correspondant par l'intermédiaire de câbles conducteurs courts 3'. Un module 6 de conditionnement des signaux SP, formant avec l'amplificateur 5 des moyens de prétraitement, assure le filtrage et la sélection des voies d'acquisition. La sélection des dérivations peut également être réalisé en amont de l'amplificateur 5.

Plusieurs chaînes d'acquisition de signaux électrophysiologiques SP différents peuvent être associées dans le dispositif capteur 1.

Simultanément et parallèlement, une mesure d'une ou de plusieurs caractéristiques du champ magnétique local est réalisée dans ledit dispositif capteur 1. Cette mesure est effectuée avantageusement en exploitant l'effet Hall au niveau d'un ou de plusieurs capteurs 7, mais d'autres techniques pourraient être utilisées. Le nombre de points de mesure, et donc de capteurs 7, du champ magnétique peut être aisément augmenté grâce à l'utilisation de la technologie microélectronique. En mesurant en deux, quatre ou six points le champ magnétique local, il est possible de déterminer, au niveau d'un module de prétraitement 8 recevant les signaux issus des capteurs 7, les gradients de champs dans une ou plusieurs, voire toutes les directions, et cela directement au niveau du ou des points de mesure 9 du signal électrophysiologique SP.

A cet effet, il est avantageux de disposer les capteurs 7 affectés à la mesure d'un gradient donné avec un espacement mutuel maximum (par exemple à proximité de bords opposés du boîtier 1' du dispositif capteur 1).

Afin de permettre une prise en compte précise des perturbations, chaque capteur 7 peut consister en un capteur de Hall sous la forme d'un circuit microélectronique en semi-conducteur CMOS standard, préférentiellement du type présentant une surface de quelques mm² (par exemple de 1 à 15 mm², préférentiellement d'environ 1 à 10 mm²) et une précision de mesure de l'ordre de 10 à 100 microteslas.

Ainsi, il est possible de mesurer des champs et variations de champs très faibles (typiquement de l'ordre du milli-tesla) par rapport au champ résident principal (de l'ordre de 0,5 T à 3 T).

De plus, le choix d'une telle solution technique à base de capteurs semiconducteurs en lieu et place des dispositifs usuels consistant en des boucles conductrices traversées par le champ magnétique, évite de parasiter les images médicales et/ou de provoquer des échauffements en cas de contact avec la peau du patient.

Pour le traitement consécutif et corrélatif des signaux de mesure SP et SM fournis respectivement par les électrodes 3 et les capteurs 7, deux solutions alternatives peuvent être envisagées :
- soit la correction des interférences magnétiques affectant les signaux électrophysiologiques SP est faite directement dans le dispositif capteur 1 par un circuit de traitement 12 formant moyen de correction (représenté en traits pointillés sur la figure 1),
- soit tous les signaux électrophysiologiques SP et de mesure des champs magnétiques SM sont transmis vers l'extérieur du dispositif capteur 1 après prétraitement et conditionnement et le traitement des interférences magnétiques est réalisé en dehors du dispositif capteur 1, dans un module 14' adapté de l'unité de gestion et de traitement.

Dans les deux variantes précitées, une modulation et une adaptation des signaux au mode d'émission sont réalisées dans un module adapté 10, 11, puis les signaux sont transmis vers le système 14 par tout mode de transmission compatible avec un environnement magnétiquement perturbé du type IRM.

Préférentiellement, la transmission du ou des signaux électrophysiologiques SP prétraités ou prétraités et corrigés et, le cas échéant, du ou des signaux de mesure SM de champ magnétique, ou de gradients de champ magnétique, est réalisée selon un mode de transmission série choisi dans le groupe formé par la transmission par fibre optique, la transmission par ondes radiofréquence et la transmission par conducteurs électriques blindés.

Au niveau de la réception de ces signaux, des moyens de conversion et de démodulation, par exemple sous forme de modules 16 adaptés de l'unité extérieure 14, traitent les signaux reçus avant leur exploitation.

Il convient de noter que le dispositif capteur 1 comprenant les moyens 4 à 12 précités est conçu de manière à avoir la consommation la plus faible possible autorisant ainsi une alimentation autonome. Un des principes de base guidant sa conception peut être l'utilisation d'une technologie microélectronique standard.

En relation avec la figure 2A des dessins annexés, on décrit ci-après à titre d'exemple une variante de réalisation du système global selon l'invention, plus particulièrement en relation avec l'acquisition de signaux ECG.

Cette figure représente, sous forme de schéma-blocs, un système 13 d'acquisition d'au moins un signal électrophysiologique SP d'un sujet 2 vivant soumis à un examen par un examen d'IRM. Ce système 13 comprend notamment une unité 14 de gestion et de traitement et au moins un dispositif capteur 1 relié à ladite unité, ledit au moins un dispositif capteur 1 étant positionné sur le sujet 2 et l'unité de traitement étant située à distance.

Le ou chaque dispositif capteur 1 (un seul est représenté sur la figure 2A), alimenté par exemple par une pile, une batterie ou une énergie lumineuse, permet le recueil de l'ECG d'un sujet 2 à l'aide de deux électrodes 3 reliées au boîtier 1' par l'intermédiaire de deux conducteurs 3', de préférence les plus courts possibles.

Un étage ou module 4, comportant au moins un amplificateur d'instrumentation et un dispositif de filtrage, formant ensemble les moyens de prétraitement, délivre le signal ECG (SP) avec le gain, la dynamique et la bande passante désirés.

Un ou deux capteurs de Hall 7 perpendiculaires entre eux, qui mesurent l'amplitude du vecteur champ magnétique qui les traverse, sont placés dans le dispositif capteur 1.

L'étage ou module 8 réalise une mise en forme des signaux SM issus des capteurs de Hall 7 avec le gain, la dynamique et la bande passante désirés (moyens de prétraitement).

Les deux ou trois signaux mesurés sont, dans des modules communs aux deux chaînes d'acquisition (10, MUX, 11), multiplexés (en MUX), puis modulés (en 10) (par exemple modulation de largeur d'impulsion) de manière à pouvoir être transmis en série sur une seule fibre optique 15 après transformation électronique/optique E/O (en 11).

La fibre optique 15 relie le dispositif capteur 1 et l'unité de traitement 14, qui peut être placée à distance.

Les signaux optiques sont restitués sous forme électronique dans un module 17 de conversion O/E et transmis à un module de traitement 14' pour y être démultiplexés, démodulés et numérisés. Le traitement pour supprimer les interférences est effectué à partir de ces données numérisées.

La synchronisation de la liaison série 15, 15' entre le ou chaque dispositif capteur 1 et l'unité de traitement 14 est assurée par une horloge 18 transmise par la fibre optique 15' après transformation électrique/optique E/O dans un module 17' adapté et utilisée après transformation optique/électrique O/E (en 11') pour la commande du modulateur 10 et du multiplexeur MUX, via le compteur 19.

Ainsi, l'unité de gestion et de traitement 14 est reliée au ou à chaque dispositif capteur 1 par une liaison série bidirectionnelle 15, 15' permettant la transmission 15 du ou de chaque signal physiologique SP recueilli par le ou les dispositif(s) capteur(s) 1, et le cas échéant ledit au moins un signal de mesure SM d'au moins une caractéristique de l'environnement magnétique au niveau du ou des points de mesure 9 du dispositif capteur 1 concerné, vers ladite unité 14 et la transmission 15' d'un signal d'horloge de cette unité 14 vers ledit ou lesdits dispositif(s) capteur(s) 1, en particulier en vue de la modulation et du multiplexage, des signaux recueillis par le ou chaque dispositif capteur 1.

Il peut être prévu que l'unité de gestion et de traitement 14 comprenne au moins un moyen d'édition du ou de chaque signal physiologique SP recueilli, par exemple un moyen d'affichage et/ou un moyen d'impression, ainsi qu'un moyen de stockage.

Il peut également être prévu que l'unité de gestion et de traitement fournisse à l'appareil IRM un signal de cadencement extrait du signal physiologique SP recueilli, ce par une liaison de transmission adaptée 20.

Les traitements pour corriger les interférences magnétiques peuvent être réalisés selon des techniques algorithmiques similaires à celles déjà connues (voir publications citées précédemment). D'autres méthodes sont cependant possibles.

Comme le montre à titre de variante avantageuse la figure 2B des dessins annexés, il peut être prévu que les capteurs 7 soient intégrés avec les modules de prétraitement 4 et 8 des signaux SP et SM, ainsi qu'avec les circuits de multiplexage MUX et de modulation 10, dans un même circuit microélectronique 21 monté dans un boîtier 1' relié aux ou portant les électrodes 3, ou encore intégré dans ces dernières.

Ce circuit 21 peut également intégrer, le cas échéant, le moyen 12 réalisant la correction du signal ou des signaux SP en fonction du signal ou des signaux SM (sous forme logicielle, matérielle ou matérielle et logicielle).

Une telle intégration, et la miniaturisation qui en résulte, permet d'aboutir à un volume très réduit pour le boîtier 1', ce qui diminue l'impact des gradients de champ sur les circuits de mesure et donc sur l'ECG recueilli, diminue la formation d'artefacts sur les images prises sous le capteur du fait de sa présence et permet donc de placer ce boîtier 1' très près des électrodes 3 même en cas d'imagerie cardiaque. Ceci autorise l'utilisation de câbles 3' très courts et contribue donc à diminuer encore plus l'impact des gradients de champ sur l'ECG. Cette diminution de volume, en permettant de placer les capteurs 7 très près des électrodes 3 de recueil du signal parasité permet également la mesure des champs magnétiques sources des parasites très près de l'endroit ou des endroits où ils agissent, ce qui est avantageux pour les traitements de correction des signaux perturbés. La miniaturisation du boîtier 1' permet d'envisager comme variante l'intégration de celui-ci dans les électrodes 3 elles-mêmes.

Ainsi, l'invention concerne un procédé, un dispositif capteur et un système d'acquisition comprenant un tel dispositif capteur, permettant de réduire, voire d'éliminer, les artefacts affectant les signaux électrophysiologiques et qui sont dus aux champs magnétiques.

Grâce à l'invention, il est donc notamment possible de réaliser une mesure locale des champs et gradients de champs magnétiques, qui permet de faire une correction plus juste des interférences liées aux gradients de champs magnétiques, ces corrections pouvant, par exemple, faire appel à des algorithmes de traitement déjà connus dans le cadre de techniques antérieures basées sur la connaissance des courants de génération des gradients de champs magnétiques.

Bien entendu, l'invention, qui porte sur un procédé, un dispositif et un système pour réduire les artefacts affectant les signaux électrophysiologiques et dus aux champs électromagnétiques, n'est pas limitée aux modes de réalisation décrits et représentés aux dessins annexés. Des modifications restent possibles, notamment du point de vue de la constitution des divers éléments ou par substitution d'équivalents techniques, sans sortir pour autant du domaine de protection de l'invention.

## Revendications

1. Procédé de recueil avec un dispositif capteur d'au moins un signal physiologique d'un sujet vivant soumis à au moins un champ électromagnétique, en particulier soumis à un environnement électromagnétique tel qu'existant dans un appareil d'investigation exploitant la résonance magnétique, consistant à réaliser une acquisition physique d'au moins un signal électrophysiologique sur le sujet, et au moins une opération de prétraitement dudit au moins un signal électrophysiologique prélevé, ce par l'intermédiaire de moyens correspondants,
procédé **caractérisé en ce qu'**il consiste également à réaliser, par l'intermédiaire de moyens additionnels (7, 8), une mesure d'au moins une caractéristique de l'environnement électromagnétique au niveau ou à proximité immédiate du ou des points ou zones (9) d'acquisition du ou des signaux électrophysiologiques (SP) sur le sujet (2), et à corriger le ou chaque signal électrophysiologique (SP) relevé et prétraité, en fonction dudit au moins un signal de mesure (SM), éventuellement prétraité, d'au moins une caractéristique de l'environnement électromagnétique du ou des points ou zones d'acquisition (9) du signal (SP) précité, caractéristique choisie dans le groupe formé par l'amplitude du vecteur champ magnétique local, dans une ou plusieurs directions, et la valeur du ou des gradients de champ magnétique présents, dans une ou plusieurs directions.

2. Procédé selon la revendication 1, **caractérisé en ce qu'**il consiste en outre à réaliser, avec des moyens adaptés (10, 11), une modulation, une adaptation au mode de transmission et une émission vers une unité de gestion et de traitement (14) distante, éventuellement associée à des moyens d'impression et/ou d'affichage, dudit au moins un signal électrophysiologique (SP) prétraité et dudit au moins un signal de mesure (SM) d'au moins une caractéristique de l'environnement électromagnétique du ou des points ou zones d'acquisition (9) du signal (SP) précité, éventuellement également prétraité, ladite unité (14) réalisant également le traitement correctif du signal (SP) en fonction du signal de mesure (SM).

3. Procédé selon la revendication 1, **caractérisé en ce qu'**il consiste, en outre, à réaliser le traitement correctif du ou de chaque signal électrophysiologique (SP) en fonction dudit au moins un signal de mesure (SM) à l'aide d'un moyen (12) adapté intégré au dispositif capteur (1) et ensuite à réaliser, avec des moyens adaptés (10, 11), une modulation, une adaptation au mode de transmission et une émission vers une unité de gestion et de traitement (14) distante, éventuellement associée à des moyens d'impression et/ou d'affichage dudit au moins un signal électrophysiologique (SP) corrigé.

4. Dispositif capteur d'au moins un signal physiologique d'un sujet vivant soumis à au moins un champ électromagnétique, en particulier soumis à un environnement électromagnétique tel qu'existant dans un appareil d'investigation exploitant la résonance magnétique, comprenant des moyens d'acquisition physique d'au moins un signal électrophysiologique sur le sujet, tels que des électrodes, et au moins des moyens de prétraitement dudit au moins un signal électrophysiologique prélevé, dispositif capteur (1) **caractérisé en ce qu'**il comprend également des moyens additionnels (7, 8) pour la mesure d'au moins une caractéristique de l'environnement électromagnétique au niveau ou à proximité du ou des points ou zones (9) d'acquisition du ou des signaux électrophysiologiques (SP) sur le sujet (2), ladite caractéristique étant choisie dans le groupe formé par l'amplitude du vecteur champ magnétique local, dans une ou plusieurs directions, et la valeur du ou des gradients de champ magnétique présents, dans une ou plusieurs directions.

5. Dispositif capteur selon la revendication 4, **caractérisé en ce que** les moyens additionnels comprennent au moins un capteur de champ magnétique (7) fournissant un signal de mesure (SM) fonction de l'amplitude du vecteur champ magnétique au niveau dudit capteur (7) et au moins un moyen (8) de prétraitement du signal délivré par le ou chaque capteur (7).

6. Dispositif capteur selon l'une quelconque des revendications 4 et 5, **caractérisé en ce qu'**il comprend plusieurs capteurs (7) de champ magnétique utilisés en combinaison, par exemple au moins une paire de capteurs (7) de champ magnétique disposés de manière espacée suivant une direction déterminée.

7. Dispositif capteur selon l'une quelconque des revendications 4 à 6, **caractérisé en ce que** les moyens additionnels comprennent au moins un capteur de champ magnétique (7) sous la forme d'un capteur à effet Hall, ou plusieurs capteurs (7) de champ magnétique sous la forme de plusieurs capteurs à effet Hall utilisés en combinaison, par exemple au moins une paire de capteurs de champ magnétique sous la forme de deux capteurs à effet Hall (7) disposés de manière espacée suivant une direction déterminée.

8. Dispositif capteur selon l'une quelconque des revendications 4 à 7, **caractérisé en ce qu'**il comprend en outre des moyens (10, 11) de modulation, d'adaptation au mode de transmission et d'émission pour ledit au moins un signal électrophysiologique (SP) prétraité et pour ledit au moins un signal de mesure (SM) d'au moins une caractéristique de l'environnement électromagnétique du ou des points ou zones d'acquisition (9) du signal (SP) précité, éventuellement également prétraité.

9. Dispositif capteur selon l'une quelconque des revendications 4 à 7, **caractérisé en ce qu'**il comprend également, d'une part, un moyen (12) de correction du ou de chaque signal électrophysiologique (SP) relevé et prétraité en fonction dudit au moins un signal de mesure (SM), éventuellement prétraité, d'au moins une caractéristique de l'environnement électromagnétique du ou des points ou zones d'acquisition (9) du signal (SP) précité, et, d'autre part, un moyen (10, 11) de modulation, d'adaptation au mode de transmission et d'émission pour ledit au moins un signal électrophysiologique (SP) corrigé.

10. Dispositif capteur selon l'une quelconque des revendications 4 à 9, **caractérisé en ce que** les moyens de prétraitement (4 ; 5, 6) dudit au moins un signal électrophysiologique (SP) relevé et les moyens additionnels (7, 8), ainsi qu'éventuellement les moyens (10, 11) de modulation et d'adaptation au mode d'émission et le moyen de correction (12), sont réalisés sous la forme de composants microélectroniques intégrés.

11. Dispositif capteur selon l'une quelconque des revendications 4 à 10, **caractérisé en ce qu'**il comprend plusieurs voies d'acquisition et de prétraitement de signaux électrophysiologiques (SP) et plusieurs voies de mesure de caractéristiques de l'environnement électromagnétique, permettant notamment la détermination des gradients de champ magnétique selon les trois axes d'un repère orthogonal attaché audit dispositif capteur (1).

12. Dispositif capteur selon l'une quelconque des revendications 8 et 9, **caractérisé en ce que** la transmission du ou des signaux électrophysiologiques (SP) prétraités ou prétraités et corrigés et, le cas échéant, du ou des signaux de mesure (SM) de champ magnétique, ou de gradients de champ magnétique, est réalisée selon un mode de transmission série choisi dans le groupe formé par la transmission par fibre optique, la transmission par ondes radiofréquence et la transmission par conducteurs électriques blindés.

13. Dispositif capteur selon l'une quelconque des revendications 5 à 7, **caractérisé en ce que** chaque capteur (7) consiste en un capteur de Hall sous la forme d'un circuit microélectronique en semi-conducteur CMOS standard, préférentiellement du type présentant une surface de quelques mm² et une précision de mesure de l'ordre de 10 à 100 microteslas.

14. Dispositif capteur selon la revendication 13, **caractérisé en ce que** les capteurs (7) sont intégrés avec les modules de prétraitement (4 et 8) des signaux (SP et SM), ainsi qu'avec les circuits de multiplexage (MUX) et de modulation (10), dans un même circuit microélectronique (21) monté dans un boîtier (1') relié aux ou portant les électrodes (3), ou encore intégré dans ces dernières.

15. Dispositif capteur selon les revendications 9 et 14, **caractérisé en ce que** le circuit (21) intègre également le moyen de correction (12).

16. Système d'acquisition d'au moins un signal électrophysiologique d'un sujet vivant soumis à un examen d'IRM, notamment pour la mise en oeuvre du procédé selon l'une quelconque des revendications 1 à 3, ce système comprenant notamment une unité de gestion et de traitement et au moins un dispositif capteur relié à ladite unité, ledit au moins un dispositif capteur étant positionné sur le sujet et l'unité de traitement étant située à distance, système (13) **caractérisé en ce que** le ou chaque dispositif capteur (1) est un dispositif capteur selon l'une quelconque des revendications 4 à 15.

17. Système selon la revendication 16, **caractérisé en ce que** l'unité de gestion et de traitement (14) est reliée au ou à chaque dispositif capteur (1) par une liaison série bidirectionnelle (15, 15') permettant la transmission (15) du ou de chaque signal physiologique (SP) recueilli par le ou les dispositif(s) capteur(s) (1), et le cas échéant ledit au moins un signal de mesure (SM) d'au moins une caractéristique de l'environnement magnétique au niveau du ou des points de mesure (9) du dispositif capteur (1) concerné, vers ladite unité (14) et la transmission (15') d'un signal d'horloge de cette unité (14) vers ledit ou lesdits dispositif(s) capteur(s) (1), en particulier en vue de la modulation et du multiplexage des signaux recueillis par le ou chaque dispositif capteur (1).

18. Système selon la revendication 16 ou 17, **caractérisé en ce que** l'unité de gestion et de traitement (14) comprend au moins un moyen d'édition du ou de chaque signal physiologique (SP) recueilli, par exemple un moyen d'affichage et/ou un moyen d'impression.

19. Système selon l'une quelconque des revendications 16 à 18, **caractérisé en ce que** l'unité de gestion et de traitement fournit à l'appareil d'IRM un signal de cadencement extrait du signal physiologique (SP) recueilli.

## Patentansprüche

1. Verfahren zur Erfassung mindestens eines physiologischen Signals eines Lebewesens, das mindestens einem elektromagnetischen Feld ausgesetzt ist, insbesondere einer elektromagnetischen Umgebung, wie sie in einem Untersuchungsgerät besteht, das mit Magnetresonanz arbeitet, mit Hilfe einer Sensorvorrichtung, darin bestehend, eine physikalische Erfassung mindestens eines elektrophysiologischen Signals am Lebewesen vorzunehmen und mindestens eine Vorbehandlung des genannten erfassten mindestens einen elektrophysiologischen Signals mit Hilfe entsprechender Mittel,
Verfahren, **dadurch gekennzeichnet, dass** es außerdem darin besteht, mit Hilfe zusätzlicher Mittel (7, 8) eine Messung mindestens einer Kenngröße der elektromagnetischen Umgebung auf dem Niveau oder in unmittelbarer Nähe des oder der Punkte oder Bereiche (9) der Erfassung des oder der elektrophysiologischen Signale (SP) am Lebewesen (2) vorzunehmen und das oder jedes erfasste und vorbehandelte elektrophysiologische Signal (SP) in Abhängigkeit vom genannten mindestens einen, gegebenenfalls vorbehandelten Messsignal (SM) mindestens einer Kenngröße des oder der Punkte oder Bereiche (9) der Erfassung des genannten Signals (SP) zu korrigieren, Kenngröße, ausgewählt aus der Gruppe, bestehend aus der Amplitude des lokalen magnetischen Feldvektors in einer oder mehreren Richtungen und dem Wert des oder der vorhandenen Magnetfeldgradienten in einer oder mehreren Richtungen.

2. Verfahren nach Patentanspruch 1, **dadurch gekennzeichnet, dass** es darin besteht, außerdem mit geeigneten Mitteln (10, 11) eine Modulation, eine Anpassung an die Übertragungsart und eine Sendung des genannten mindestens einen vorbehandelten elektrophysiologischen Signals (SP) und des genannten mindestens einen, gegebenenfalls ebenfalls vorbehandelten, Messsignals (SM) mindestens einer Kenngröße der elektromagnetischen Umgebung des oder der Punkte oder Bereiche der Erfassung (9) des genannten Signals (SP) zu einer fernen Steuer- und Verarbeitungseinheit (14) vorzunehmen, die gegebenenfalls mit Druck- und/oder Anzeigemitteln verbunden ist, wobei die genannte Einheit (14) ebenfalls die Korrektur des Signals (SP) in Abhängigkeit vom Messsignal (SM) vornimmt.

3. Verfahren nach Patentanspruch 1, **dadurch gekennzeichnet, dass** es außerdem darin besteht, die Korrektur des oder jedes elektrophysiologischen Signals (SP) in Abhängigkeit vom genannten mindestens einen Messsignal (SM) mit Hilfe eines geeigneten Mittels (12) vorzunehmen, das in die Sensorvorrichtung (1) eingebaut ist, und danach mit geeigneten Mitteln (10, 11) eine Modulation, eine Anpassung an die Übertragungsart und eine Sendung zu einer fernen Steuer- und Verarbeitungseinheit (14) vorzunehmen, die gegebenenfalls mit Druck- und/oder Anzeigemitteln des genannten mindestens einen korrigierten elektrophysiologischen Signals (SP) verbunden ist.

4. Sensorvorrichtung mindestens eines physiologischen Signals eines Lebewesens, das mindestens einem elektromagnetischen Feld ausgesetzt ist, insbesondere einer elektromagnetischen Umgebung, wie sie in einem Untersuchungsgerät besteht, das mit Magnetresonanz arbeitet, Mittel zur physikalischen Erfassung mindestens eines elektrophysiologischen Signals am Lebewesen, wie etwa Elektroden, umfassend und mindestens Mittel zur Vorbehandlung des genannten erfassten mindestens einen elektrophysiologischen Signals, Sensorvorrichtung (1), **dadurch gekennzeichnet, dass** sie auch zusätzliche Mittel (7, 8) zur Messung mindestens einer Kenngröße der elektromagnetischen Umgebung auf dem Niveau oder in unmittelbarer Nähe des oder der Punkte oder Bereiche (9) der Erfassung des oder der elektrophysiologischen Signals/e (SP) am Lebewesen (2) umfasst, wobei die genannte Kenngröße aus der Gruppe ausgewählt wird, bestehend aus der Amplitude des lokalen magnetischen Feldvektors in einer oder mehreren Richtungen und dem Wert des oder der vorhandenen Magnetfeldgradienten in einer oder mehreren Richtungen.

5. Sensorvorrichtung nach Patentanspruch 4, **dadurch gekennzeichnet, dass** die zusätzlichen Mittel mindestens einen Magnetfeldsensor (7) umfassen, der ein Messsignal (SM) in Abhängigkeit von der Amplitude des magnetischen Feldvektors auf dem Niveau des genannten Sensors (7) ausgibt, und mindestens ein Mittel (8) zur Vorbehandlung des von dem oder jedem Sensor (7) ausgegebenen Signals.

6. Sensorvorrichtung nach irgendeinem der Patentansprüche 4 und 5, **dadurch gekennzeichnet, dass** sie mehrere Magnetfeldsensoren (7) umfasst, die kombiniert verwendet werden, beispielsweise mindestens ein Paar Magnetfeldsensoren (7), die in einer festgelegten Richtung im Abstand voneinander angeordnet sind.

7. Sensorvorrichtung nach irgendeinem der Patentansprüche 4 bis 6, **dadurch gekennzeichnet, dass** die zusätzlichen Mittel mindestens einen Magnetfeldsensor (7) in Form eines Hall-Effekt-Sensors umfassen oder mehrere Magnetfeldsensoren (7) in Form mehrerer Hall-Effekt-Sensoren, die kombiniert verwendet werden, beispielsweise mindestens ein Paar Magnetfeldsensoren in Form von zwei Hall-Effekt-Sensoren (7), die in einer festgelegten Richtung im Abstand voneinander angeordnet sind.

8. Sensorvorrichtung nach irgendeinem der Patentansprüche 4 bis 7, **dadurch gekennzeichnet, dass** sie außerdem Mittel (10, 11) zur Modulation, Anpassung an die Übertragungsart und Sendung des genannten mindestens einen vorbehandelten elektrophysiologischen Signals (SP) und des genannten mindestens einen, gegebenenfalls ebenfalls vorbehandelten, Messsignals (SM) mindestens einer Kenngröße der elektromagnetischen Umgebung des oder der Punkte oder Bereiche der Erfassung (9) des genannten Signals (SP), umfasst.

9. Sensorvorrichtung nach irgendeinem der Patentansprüche 4 bis 7, **dadurch gekennzeichnet, dass** sie außerdem einerseits ein Mittel (12) zur Korrektur des oder jedes erfassten und vorbehandelten elektrophysiologischen Signals (SP) in Abhängigkeit vom genannten gegebenenfalls vorbehandelten Messsignal (SM) mindestens einer Kenngröße der elektromagnetischen Umgebung des oder der Punkte oder Bereiche der Erfassung (9) des genannten Signals (SP) umfasst und andererseits ein Mittel (10, 11) zur Modulation, Anpassung an die Übertragungsart und Sendung des genannten mindestens einen korrigierten elektrophysiologischen Signals (SP).

10. Sensorvorrichtung nach irgendeinem der Patentansprüche 4 bis 9, **dadurch gekennzeichnet, dass** die Mittel zur Vorbehandlung (4; 5, 6) des genannten erfassten mindestens einen elektrophysiologischen Signals (SP) und die zusätzlichen Mittel (7, 8), sowie gegebenenfalls die Mittel (10, 11) zur Modulation und Anpassung an die Übertragungsart und das Korrekturmittel (12) in Form integrierter mikroelektronischer Bauteile ausgeführt sind.

11. Sensorvorrichtung nach irgendeinem der Patentansprüche 4 bis 10, **dadurch gekennzeichnet, dass** sie mehrere Kanäle zur Erfassung und Vorbehandlung elektrophysiologischer Signale (SP) umfasst und mehrere Kanäle zur Messung von Kenngrößen der elektromagnetischen Umgebung, was insbesondere die Bestimmung von Gradienten des magnetischen Feldes längs den drei Achsen eines rechtwinkligen Koordinatensystems ermöglicht, das mit der genannten Sensorvorrichtung (1) verbunden ist.

12. Sensorvorrichtung nach irgendeinem der Patentansprüche 8 und 9, **dadurch gekennzeichnet, dass** die Übertragung des oder der vorbehandelten oder vorbehandelten und korrigierten elektrophysiologischen Signale (SP) und gegebenenfalls des oder der Messsignale (SM) des Magnetfeldes oder von Gradienten des Magnetfeldes, in einer seriellen Übertragungsart erfolgt, ausgewählt aus der Gruppe, bestehend aus der Übertragung über Lichtleiter, der Übertragung über Radiowellen und der Übertragung über abgeschirmte elektrische Leiter.

13. Sensorvorrichtung nach irgendeinem der Patentansprüche 5 bis 7, **dadurch gekennzeichnet, dass** jeder Sensor (7) aus einem Hall-Sensor in Form einer mikroelektronischen Standard-CMOS-Halbleiterschaltung besteht, vorzugsweise des Typs, der eine Oberfläche von einigen mm² aufweist und eine Messgenauigkeit in der Größenordnung von 10 bis 100 Mikrotesla.

14. Sensorvorrichtung nach Patentanspruch 13, **dadurch gekennzeichnet, dass** die Sensoren (7) mit den Vorbehandlungsmodulen (4 und 8) der Signale (SP und SM), sowie mit den Multiplexerschaltungen (MUX) und den Modulationsschaltungen (10) in ein und derselben mikroelektronischen Schaltung (21) integriert sind, die in einem Gehäuse (1') montiert ist, das mit den Elektroden (3) verbunden ist oder diese trägt oder auch in diese eingebaut ist.

15. Sensorvorrichtung nach den Patentansprüchen 9 und 14, **dadurch gekennzeichnet, dass** die Schaltung (21) ebenfalls das Korrekturmittel (12) enthält.

16. System zur Erfassung mindestens eines elektrophysiologischen Signals eines Lebewesens, an dem eine NMR-Untersuchung vorgenommen wird, insbesondere zur Ausführung des Verfahrens nach irgendeinem der Patentansprüche 1 bis 3, wobei dieses System insbesondere eine Steuer- und Verarbeitungseinheit umfasst und mindestens eine Sensorvorrichtung, die mit der genannten Einheit verbunden ist, wobei die genannte mindestens eine Sensorvorrichtung am Lebewesen angeordnet ist und sich die Verarbeitungseinheit fern davon befindet, System (13), **dadurch gekennzeichnet, dass** die oder jede Sensorvorrichtung (1) eine Sensorvorrichtung nach irgendeinem der Patentansprüche 4 bis 15 ist.

17. System nach Patentanspruch 16, **dadurch gekennzeichnet, dass** die Steuer- und Verarbeitungseinheit (14) über eine serielle Zweirichtungsverbindung (15, 15') mit der oder jeder Sensorvorrichtung (1) verbunden ist, die die Übertragung (15) des oder jedes von der oder den Sensorvorrichtung(en) (1) erfassten physiologischen Signals (SP) und gegebenenfalls des genannten mindestens einen Messsignals (SM) mindestens einer Kenngröße der magnetischen Umgebung auf dem Niveau des oder der Messpunkte (9) der betreffenden Sensorvorrichtung (1) zur genannten Einheit (14) erlaubt und die Übertragung (15') eines Taktsignals dieser Einheit (14) zu der oder den genannten Sensorvorrichtung(en) (1), insbesondere zur Modulation und zum Multiplexen der durch die oder jede Sensorvorrichtung (1) erfassten Signale.

18. System nach Patentanspruch 16 oder 17, **dadurch gekennzeichnet, dass** die Steuer- und Verarbeitungseinheit (14) mindestens ein Ausgabemittel des oder jedes erfassten physiologischen Signals (SP) umfasst, beispielsweise ein Anzeige- und/oder ein Druckmittel.

19. System nach irgendeinem der Patentansprüche 16 bis 18, **dadurch gekennzeichnet, dass** die Steuer- und Verarbeitungseinheit an das NMR-Gerät ein Wiederholungsratensignal abgibt, das aus dem erfassten physiologischen Signal (SP) abgeleitet wurde.

## Claims

1. Process for collecting, with a sensor device, at least one physiological signal from a living subject who is subjected to at least one electromagnetic field, in particular subjected to an electromagnetic environment such as the one existing in an investigative device that uses magnetic resonance, consisting in implementing, via corresponding means, a physical acquisition of at least one electrophysiological signal on the subject, and at least one pre-processing operation of said at least one electrophysiological signal that is detected,
process that is **characterised in that** it also consists in producing, using additional means (7, 8), a measurement of at least one characteristic of the electromagnetic environment at or in the immediate proximity of the point or points or zones (9) for acquisition of the electrophysiological signal(s) (SP) on the subject (2), and in correcting the - or each - electrophysiological signal (SP) that is detected and pre-processed, based on said at least one measuring signal (SM), optionally pre-processed, of at least one characteristic of the electromagnetic environment of the point or points or zones (9) for acquisition of the above-mentioned signal (SP), a characteristic that is selected from the group that is formed by the amplitude of the local magnetic field vector, in one or more directions, and the value of the magnetic field gradient(s) present, in one or more directions.

2. Process according to claim 1, **characterised in that** it also consists in carrying out, with suitable means (10, 11), a modulation, an adaptation to the transmission mode and an emission to a unit for control and remote processing (14), optionally combined with means for printing and/or display, said at least one pre-processed electrophysiological signal (SP) and said at least one measuring signal (SM) of at least one characteristic of the electromagnetic environment of the point or points or zones (9) for acquisition of the above-mentioned signal (SP), optionally also pre-processed, whereby said unit (14) also carries out the corrective processing of the signal (SP) based on the measuring signal (SM).

3. Process according to claim 1, **characterised in that** it also consists in carrying out the corrective processing of the - or of each - electrophysiological signal (SP) based on said at least one measuring signal (SM) using a suitable means (12) that is integrated with the sensor device (1) and then in carrying out, with suitable means (10,11), a modulation, an adaptation to the transmission mode, and an emission to a unit for control and remote processing (14), optionally combined with means for printing and/or display of said at least one corrected electrophysiological signal (SP).

4. Sensor device of at least one physiological signal of a living subject who is subjected to at least one electromagnetic field, in particular subjected to an electromagnetic environment such as the one existing in an investigative device that uses magnetic resonance, comprising means for physical acquisition of at least one electrophysiological signal on the subject, such as electrodes, and at least pre-processing means of said at least one electrophysiological signal that is detected, a sensor device (1) that is **characterised in that** it also comprises additional means (7, 8) for the measurement of at least one characteristic of the electromagnetic environment at or in the proximity of the point or points or zones (9) for acquisition of the electrophysiological signal(s) (SP) on the subject (2), whereby said characteristic is selected from the group that is formed by the amplitude of the local magnetic field vector, in one or more directions, and the value of the magnetic field gradient(s) present, in one or more directions.

5. Sensor device according to claim 4, **characterised in that** the additional means comprise at least one magnetic field sensor (7) that provides a measuring signal (SM) that is based on the amplitude of the magnetic field vector at said sensor (7) and at least one means (8) for pre-processing the signal that is delivered by the - or each - sensor (7).

6. Sensor device according to any of claims 4 and 5, **characterised in that** it comprises several magnetic field sensors (7) that are used in combination, for example at least one pair of magnetic field sensors (7) that are arranged in a spaced manner in a determined direction.

7. Sensor device according to any of claims 4 to 6, **characterised in that** the additional means comprise at least one magnetic field sensor (7) in the form of a Hall effect sensor, or several magnetic field sensors (7) in the form of several Hall effect sensors that are used in combination, for example at least one pair of magnetic field sensors in the form of two Hall effect sensors (7) that are arranged in a spaced manner in a determined direction.

8. Sensor device according to any of claims 4 to 7, **characterised in that** it also comprises means (10, 11) for modulation, adaptation to the mode of transmission and emission for said at least one pre-processed electrophysiological signal (SP) and for said at least one measuring signal (SM) of at least one characteristic of the electromagnetic environment of the point or points or zones (9) for acquisition of the above-mentioned signal (SP), optionally also pre-processed.

9. Sensor device according to any of claims 4 to 7, **characterised in that** it also comprises, on the one hand, a means (12) for correction of the - or of each - electrophysiological signal (SP) that is detected and pre-processed based on said at least one measuring signal (SM), optionally pre-processed, of at least one characteristic of the electromagnetic environment of the point or points or zones (9) for acquisition of the above-mentioned signal (SP), and, on the other hand, a means (10, 11) for modulation, adaptation to the mode of transmission and emission for said at least one corrected electrophysiological signal (SP).

10. Sensor device according to any of claims 4 to 9, **characterised in that** the means (4; 5, 6) for pre-processing said at least one electrophysiological signal (SP) that is detected and the additional means (7, 8), as well as optionally the means (10, 11) for modulation and adaptation to the mode of emission and the means for correction (12), are produced in the form of integrated microelectronic components.

11. Sensor device according to any of claims 4 to 10, **characterised in that** it comprises several channels for acquisition and pre-processing of electrophysiological signals (SP) and several channels for measuring characteristics of the electromagnetic environment, making possible in particular the determination of the magnetic field gradients along the three axes of an orthogonal reference that is attached to said sensor device (1).

12. Sensor device according to any of claims 8 and 9, **characterised in that** the transmission of the electrophysiological signal(s) (SP) that are pre-processed or pre-processed and corrected, and, if necessary, the signal(s) (SM) for measuring the magnetic field, or magnetic field gradients, is carried out according to a serial transmission mode that is selected from the group that is formed by the transmission by optical fibers, the transmission by radiofrequency waves, and the transmission by shielded electrical conductors.

13. Sensor device according to any of claims 5 to 7, **characterised in that** each sensor (7) consists of a Hall sensor in the form of a microelectronic circuit of a standard CMOS semiconductor, preferably of the type that has a surface area of several mm² and a measurement precision on the order of 10 to 100 microtesla.

14. Sensor device according to claim 13, **characterised in that** the sensors (7) are integrated with the modules (4 and 8) for pre-processing the signals (SP and SM), as well as with circuits for multiplexing (MUX) and modulation (10), in the same microelectronic circuit (21) that is mounted in a housing (1') that is connected to or that carries the electrodes (3), or else integrated in the latter.

15. Sensor device according to claims 9 and 14, **characterised in that** the circuit (21) also integrates the correction means (12).

16. System for acquisition of at least one electrophysiological signal of a living subject who is subjected to an MRI examination, in particular for the implementation of the process according to any of claims 1 to 3, whereby this system comprises in particular a unit for control and for processing and at least one sensor device that is connected to said unit, whereby said at least one sensor device is positioned on the subject, and the processing unit is located at a distance, a system (13) **characterised in that** the - or each - sensor device (1) is a sensor device according to any of claims 4 to 15.

17. System according to claim 16, **characterised in that** the unit (14) for control and processing is connected to the - or to each - sensor device (1) by a bidirectional serial connection (15, 15') that makes possible the transmission (15) of the - or of each - physiological signal (SP) that is collected by the sensor device(s) (1), and, if necessary, said at least one measuring signal (SM) of at least one characteristic of the magnetic environment at the measuring point(s) (9) of the sensor device (1) that is involved, to said unit (14) and the transmission (15') of a clock signal from this unit (14) to said sensor device(s) (1), in particular for the purpose of modulation and multiplexing of signals that are collected by the - or each - sensor device (1).

18. System according to claim 16 or 17, **characterised in that** the unit (14) for control and processing comprises at least one means for editing the - or each - physiological signal (SP) that is collected, for example, a display means and/or a printing means.

19. System according to any of claims 16 to 18, **characterised in that** the unit for control and processing provides to the MRI device a sequencing signal that is extracted from the physiological signal (SP) that is collected.
